# EUROPÄISCHE PATENTSCHRIFT

(11) **EP 2 991 974 B1**
(45) Veröffentlichungstag und Bekanntmachung des Hinweises auf die Patenterteilung: **11.04.2018**
(21) Anmeldenummer: 14720115.6
(22) Anmeldetag: 28.04.2014
(51) Int. Cl.: C07D 309/10, C11B 9/00

(54) **VERFAHREN ZUR HERSTELLUNG VON 2-SUBSTITUIERTEN 4-HYDROXY-4-METHYL-TETRAHYDROPYRANEN IN EINER REAKTORKASKADE**
PROCESS FOR THE PREPARATION OF 2-SUBSTITUTED 4-HYDROXY-4-METHYL-TETRAHYDROPYRANS IN A REACTOR CASCADE
PROCÉDÉ DE PRODUCTION DE 4-HYDROXY-4-MÉTHYL-TÉTRAHYDROPYRANES 2-SUBSTITUÉS DANS UNE CASCADE DE RÉACTEURS

(30) Priorität: 29.04.2013 EP 13165767
(43) Veröffentlichungstag der Anmeldung: 09.03.2016
(73) Patentinhaber: BASF SE, 67056 Ludwigshafen am Rhein (DE)
(72) Erfinder: STORK, Timon, 68163 Mannheim (DE); BECK, Karl, 76684 Östringen (DE); EBEL, Klaus, 68542 Heddesheim (DE); BEY, Oliver, 67150 Niederkirchen (DE); GRALLA, Gabriele, 68161 Mannheim (DE)
(86) Internationale Anmeldenummer: PCT/EP2014/058538
(87) Internationale Veröffentlichungsnummer: WO 2014/177486

(56) Entgegenhaltungen:
- WO-A1-2010/133473
- US-A1- 2011 295 024

## Beschreibung

### HINTERGRUND DER ERFINDUNG

Die vorliegende Erfindung betrifft ein Verfahren zur Herstellung von 2-substituierten 4-Hydroxy-4-methyl-tetrahydropyranen.

### STAND DER TECHNIK

2-substituierte 4-Hydroxy-4-methyl-tetrahydropyrane sind wertvolle Verbindungen für den Einsatz als Aromachemikalien. So zeichnet sich beispielsweise das cis/trans-Diastereomerengemisch des 2-(2-Methylpropyl)-4-hydroxy-4-methyl-tetrahydropyrans 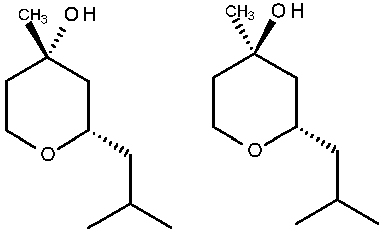 durch einen angenehmen Maiglöckchenduft aus und ist in besonderem Maße zur Verwendung als Aromachemikalie, z. B. zur Herstellung von Riechstoffkompositionen, geeignet.

Die EP 1 493 737 A1 offenbart ein Verfahren zur Herstellung von Gemischen von ethylenisch ungesättigten 4-Methyl- bzw. 4-Methylenpyranen und den entsprechenden 4-Hydroxypyranen durch Umsetzung der entsprechenden Aldehyde mit Isoprenol, wobei die Umsetzung in einem Reaktionssystem initiiert wird, in dem das molare Verhältnis von Aldehyd zu Isoprenol größer als 1 ist, d. h. der Aldehyd im Überschuss eingesetzt wird. Darüber hinaus offenbart das Dokument die anschließende Dehydratisierung der genannten Gemische zu den gewünschten ethylenisch ungesättigten Pyranen. Als geeignete Katalysatoren für den ersten Reaktionsschritt werden Mineralsäuren wie Salzsäure oder Schwefelsäure, bevorzugt jedoch Methansulfonsäure oder p-Toluolsulfonsäure genannt.

EP 1 516 879 A1 offenbart ein Verfahren zur Herstellung von ethylenisch ungesättigten 4-Methyl- bzw. 4-Methylenpyranen durch Umsetzung eines entsprechenden Aldehyds mit Isoprenol unter dehydratisierenden Bedingungen, wobei die Wassermenge im Reaktor bis zu 0,25 Gew.-% beträgt, während der Umsatz der im Unterschuss eingesetzten Ausgangsverbindung weniger als 50 % beträgt. Als hierfür geeignete Katalysatoren werden ebenfalls Mineralsäuren wie Salzsäure oder Schwefelsäure, bevorzugt jedoch Methansulfonsäure oder p-Toluolsulfonsäure genannt.

Die WO 2010/133473 beschreibt ein Verfahren zur Herstellung von 2-substituierten 4-Hydroxy-4-methyl-tetrahydropyranen der Formel (I) 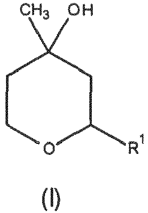 wobei der Rest R¹ für einen geradkettigen oder verzweigten Alkyl- oder Alkenylrest mit 1 bis 12 Kohlenstoffatomen, einen gegebenenfalls Alkyl-substituierten Cycloalkylrest mit insgesamt 3 bis 12 Kohlenstoffatomen oder einen gegebenenfalls Alkyl- und/oder Alkoxy-substituierten Arylrest mit insgesamt 6 bis 12 Kohlenstoffatomen steht, bei dem man Isoprenol (3-Methylbut-3-en-1-ol) mit einem Aldehyd der Formel R¹-CHO umsetzt, wobei man die Umsetzung in Gegenwart von Wasser und in Gegenwart eines stark sauren Kationenaustauschers durchführt.

Die WO 2011/154330 beschreibt ein zur WO 2010/133473 vergleichbares Verfahren, wobei das erhaltene Reaktionsgemisch einer destillativen Aufarbeitung in einer Trennwandkolonne oder in zwei thermisch gekoppelten Destillationskolonnen zugeführt wird.

Die US 2011/295024 A1 beschreibt ein kontinuierliches Verfahren zur Herstellung von 2-substituierten 4-Hydroxy-4-methyl-tetrahydropyranen aus 3-Methylbut-3-en-1-ol und einem Aldehyd in Gegenwart eines sauren Ionentauschers und Wasser.

Die unveröffentlichte europäische Patentanmeldung 12188518.0 beschreibt ein Verfahren zur Herstellung von 2-substituierten 4-Hydroxy-4-methyl-tetrahydropyranen der allgemeinen Formel (I) und von 2-substituierten 4-Methyl-tetrahydropyranen der allgemeinen Formel (II) 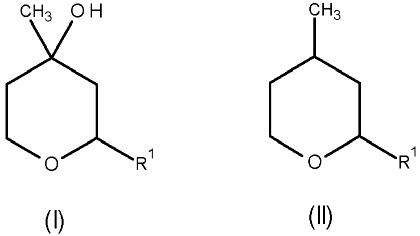 worin
- R¹: für geradkettiges oder verzweigtes C₁-C₁₂-Alkyl, geradkettiges oder verzweigtes C₂-C₁₂-Alkenyl, unsubstituiertes oder mit C₁-C₁₂-Alkyl und/oder C₁-C₁₂-Alkoxy substituiertes Cycloalkyl mit insgesamt 3 bis 20 Kohlenstoffatomen oder unsubstituiertes oder mit C₁-C₁₂-Alkyl und/oder C₁-C₁₂-Alkoxy substituiertes Aryl mit insgesamt 6 bis 20 Kohlenstoffatomen steht,
bei dem man
a) 3-Methylbut-3-en-1-ol der Formel (III) 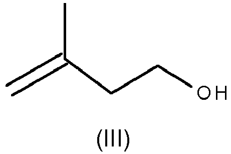 mit einem Aldehyd der Formel (IV)

   R¹-CHO (IV)

   wobei R¹ in der Formel (IV) die zuvor angegebene Bedeutung hat,
   in Gegenwart eines sauren Katalysators umsetzt, wobei ein Reaktionsgemisch erhalten wird, das wenigstens ein 2-substituiertes 4-Hydroxy-4-methyl-tetrahydropyran der allgemeinen Formel (I), wenigstens eine der Verbindungen (V.1), (V.2) oder (V.3) und wenigstens eine Dioxanverbindung (VI) enthält 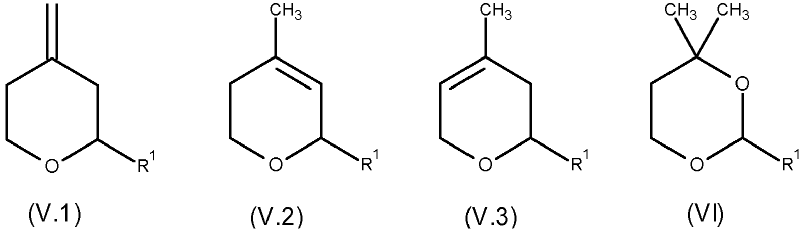 wobei R¹ in der Formel (VI) die zuvor angegebene Bedeutung hat,
b) das Reaktionsprodukt aus Schritt a) einer Auftrennung unter Erhalt einer an 2-substituierten 4-Hydroxy-4-methyl-tetrahydropyranen der allgemeinen Formel (I) angereicherten Fraktion und einer Fraktion, die wenigstens eine der Verbindungen (V.1), (V.2) oder (V.3) und wenigstens eine Dioxanverbindung (VI) enthält, unterzieht,
c) die Fraktion, die wenigstens eine der Verbindungen (V.1), (V.2) oder (V.3) und wenigstens eine Dioxanverbindung (VI) enthält, einer Hydrierung unterzieht,
d) aus dem in Schritt c) erhaltenen Hydrierprodukt eine an 2-substituierten 4-Methyl-tetrahydropyranen (II) angereicherte Fraktion und eine an der wenigstens einen Dioxanverbindung (VI) angereicherte Fraktion isoliert.

Der vorliegenden Erfindung liegt die Aufgabe zugrunde, ein verbessertes Verfahren zur Herstellung 2-substituierter 4-Hydroxy-4-methyl-tetrahydropyrane zur Verfügung zu stellen, das eine effektive Herstellung in technischem Maßstab unter möglichst geringer Bildung unerwünschter und zu entsorgender Nebenprodukte ermöglicht.

Überraschenderweise wurde jetzt gefunden, dass diese Aufgabe durch eine Fahrweise unter Einsatz von wenigstens zwei in Reihe geschalteten Reaktoren gelöst wird. Dabei handelt es sich speziell um ein kontinuierliches Verfahren.

### ZUSAMMENFASSUNG DER ERFINDUNG

Gegenstand der Erfindung ist ein Verfahren zur Herstellung von 2-substituierten 4-Hydroxy-4-methyl-tetrahydropyranen der allgemeinen Formel (I) 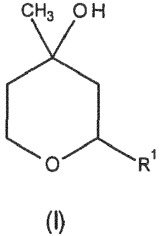 worin
- R¹: für geradkettiges oder verzweigtes C₁-C₁₂-Alkyl, geradkettiges oder verzweigtes C₂-C₁₂-Alkenyl, unsubstituiertes oder mit C₁-C₁₂-Alkyl und/oder C₁-C₁₂-Alkoxy substituiertes Cycloalkyl mit insgesamt 3 bis 20 Kohlenstoffatomen oder unsubstituiertes oder mit C₁-C₁₂-Alkyl und/oder C₁-C₁₂-Alkoxy substituiertes Aryl mit insgesamt 6 bis 20 Kohlenstoffatomen steht,
umfassend eine Umsetzung von 3-Methylbut-3-en-1-ol der Formel (III) 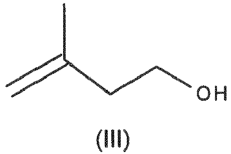 mit einem Aldehyd der Formel (IV)

R1-CHO (IV)

wobei R¹ in der Formel (IV) die zuvor angegebene Bedeutung hat,
in Gegenwart eines sauren Katalysators, dadurch gekennzeichnet, dass die Umsetzung in einer Anordnung aus n in Reihe geschalteter Reaktoren erfolgt, wobei n für eine natürliche Zahl von wenigstens 2 steht, wobei zwischen dem in Strömungsrichtung ersten und letzten Reaktor einen Teilstrom entnommen und in einen stromaufwärts der Entnahmestelle gelegenen Reaktor eingespeist wird.

### BESCHREIBUNG DER ERFINDUNG

Das erfindungsgemäße Verfahren weist die folgenden Vorteile auf:
- Das erfindungsgemäße Verfahren ermöglicht eine geringere thermische Belastung des Reaktorinhalts durch eine geringere Maximaltemperatur und/oder die Vermeidung von Temperaturspitzen.
- Das Verfahren ermöglicht somit höhere Ausbeuten und/oder eine höhere Selektivität bezüglich der Zielverbindungen.
- Eine geringere Maximaltemperatur und/oder die Vermeidung von Temperaturspitzen sind auch sicherheitstechnisch vorteilhaft und/oder ermöglichen eine längere Katalysatorstandzeit.
- Speziell der Einsatz eines Katalysatorfestbetts kann sich zusätzlich vorteilhaft auf die Katalysatorstandzeit auswirken. Somit werden langwierige An- und Abfahrvorgänge zum Tausch von verbrauchtem Katalysator bzw. zur Katalysatorregenerierung vermieden. Zudem verringert der Einsatz eines Katalysatorfestbetts auch die mechanische Belastung und Zerstörung des Katalysators.

Sofern im Folgenden nicht genauer angegeben, bezeichnen die Begriffe "2-substituiertes 4-Hydroxy-4-methyl-tetrahydropyran" und "2-(2-Methylpropyl)-4-hydroxy-4-methyl-tetrahydropyran" im Rahmen der Erfindung cis/trans-Gemische jedweder Zusammensetzung sowie die reinen Konformations-Isomere. Die zuvor genannten Begriffe bezeichnen weiterhin alle Enantiomere in Reinform sowie racemische und optisch aktive Gemische der Enantiomeren dieser Verbindungen.

Im Rahmen der vorliegenden Erfindung steht der Ausdruck geradkettiges oder verzweigtes Alkyl vorzugsweise für C₁-C₆-Alkyl und besonders bevorzugt für C₁-C₄-Alkyl. Alkyl steht insbesondere für Methyl, Ethyl, n-Propyl, Isopropyl, n-Butyl, Isobutyl (2-Methylpropyl), sec.-Butyl (1-Methylpropyl), tert.-Butyl (1,1-Dimethylethyl), n-Pentyl oder n-Hexyl. Speziell steht Alkyl für Methyl, Ethyl, n-Propyl, Isopropyl, oder Isobutyl.

Im Rahmen der vorliegenden Erfindung steht der Ausdruck geradkettiges oder verzweigtes Alkoxy vorzugsweise für C₁-C₆-Alkoxy und besonders bevorzugt für C₁-C₄-Alkoxy. Alkoxy steht insbesondere für Methoxy, Ethoxy, n-Propyloxy, Isopropyloxy, n-Butyloxy, Isobutyloxy, sec.-Butyloxy, tert.-Butyloxy, n-Pentyloxy oder n-Hexyloxy. Speziell steht Alkoxy für Methoxy, Ethoxy, n-Propyloxy, Isopropyloxy, oder Isobutyloxy.

Im Rahmen der vorliegenden Erfindung steht der Ausdruck geradkettiges oder verzweigtes Alkenyl vorzugsweise für C₂-C₆-Alkenyl und besonders bevorzugt für C₂-C₄-Alkenyl. Der Alkenylrest weist neben Einfachbindungen noch eine oder mehrere, bevorzugt 1 bis 3, besonders bevorzugt 1 oder 2 und ganz besonders bevorzugt eine ethylenische Doppelbindung auf. Alkenyl steht insbesondere für Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl oder 2-Methyl-2-propenyl.

Im Rahmen der Erfindung bezeichnet Cycloalkyl einen cycloaliphatischen Rest mit vorzugsweise 3 bis 10, besonders bevorzugt 5 bis 8, Kohlenstoffatomen. Beispiele für Cycloalkylgruppen sind insbesondere Cyclopropyl, Cyclobutyl, Cyclopentyl, Cyclohexyl, Cycloheptyl oder Cyclooctyl. Speziell steht Cycloalkyl für Cyclohexyl.

Substituierte Cycloalkylgruppen können in Abhängigkeit von der Ringgröße einen oder mehrere (z. B. 1, 2, 3, 4 oder 5) Substituenten aufweisen. Diese sind vorzugsweise unabhängig voneinander ausgewählt unter C₁-C₆-Alkyl und C₁-C₆-Alkoxy. Die Cycloalkylgruppen tragen im Falle einer Substitution vorzugsweise eine oder mehrere, beispielsweise eine, zwei, drei, vier oder fünf C₁-C₆-Alkylgruppen. Beispiele für substituierte Cycloalkylgruppen sind insbesondere 2- und 3-Methylcyclopentyl, 2- und 3-Ethylcyclopentyl, 2-, 3- und 4-Methylcyclohexyl, 2-, 3- und 4-Ethylcyclohexyl, 2-, 3- und 4-Propylcyclohexyl, 2-, 3- und 4-Isopropylcyclohexyl, 2-, 3- und 4-Butylcyclohexyl und 2-, 3- und 4-Isobutylcyclohexyl.

Der Ausdruck "Aryl" umfasst im Rahmen der vorliegenden Erfindung ein- oder mehrkernige aromatische Kohlenwasserstoffreste mit üblicherweise 6 bis 18, vorzugsweise 6 bis 14, besonders bevorzugt 6 bis 10 Kohlenstoffatomen. Beispiele für Aryl sind insbesondere Phenyl, Naphthyl, Indenyl, Fluorenyl, Anthracenyl, Phenanthrenyl, Naphthacenyl, Chrysenyl, Pyrenyl, etc., und speziell Phenyl oder Naphthyl.

Substituierte Aryle können in Abhängigkeit von der Anzahl und Größe ihrer Ringsysteme einen oder mehrere (z. B. 1, 2, 3, 4 oder 5) Substituenten aufweisen. Diese sind vorzugsweise unabhängig voneinander ausgewählt unter C₁-C₆-Alkyl und C₁-C₆-Alkoxy. Beispiele für substituierte Arylreste sind 2-, 3- und 4-Methylphenyl, 2,4-, 2,5-, 3,5- und 2,6-Dimethylphenyl, 2,4,6-Trimethylphenyl, 2-, 3- und 4-Ethylphenyl, 2,4-, 2,5-, 3,5- und 2,6-Diethylphenyl, 2,4,6-Triethylphenyl, 2-, 3- und 4-Propylphenyl, 2,4-, 2,5-, 3,5- und 2,6-Dipropylphenyl, 2,4,6-Tripropylphenyl, 2-, 3- und 4-Isopropylphenyl, 2,4-, 2,5-, 3,5- und 2,6-Diisopropylphenyl, 2,4,6-Triisopropylphenyl, 2-, 3- und 4-Butylphenyl, 2,4-, 2,5-, 3,5- und 2,6-Dibutylphenyl, 2,4,6-Tributylphenyl, 2-, 3- und 4-Isobutylphenyl, 2,4-, 2,5-, 3,5- und 2,6-Diisobutylphenyl, 2,4,6-Triisobutylphenyl, 2-, 3- und 4-sec-Butylphenyl, 2,4-, 2,5-, 3,5- und 2,6-Di-sec-butylphenyl, 2,4,6-Tri-sec-butylphenyl, 2-, 3- und 4-tert.-Butylphenyl, 2,4-, 2,5-, 3,5- und 2,6-Di-tert.-butylphenyl und 2,4,6-Tri-tert.-butylphenyl.

Einer der Ausgangsstoffe für das erfindungsgemäße Verfahren ist 3-Methylbut-3-en-1-ol (Isoprenol) der Formel (III),

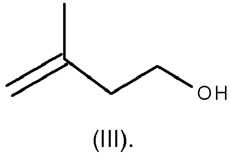

Isoprenol ist nach bekannten Verfahren aus Isobuten und Formaldehyd in jedem Maßstab gut zugänglich und kommerziell verfügbar. An die Reinheit, Qualität oder Herstellverfahren des erfindungsgemäß einzusetzenden Isoprenols sind keine besonderen Anforderungen zu stellen. Es kann in handelsüblicher Qualität und Reinheit in das erfindungsgemäße Verfahren eingesetzt werden. Bevorzugt setzt man Isoprenol ein, das eine Reinheit von 90 Gew.-% oder darüber hat, besonders bevorzugt solches mit einer Reinheit von 95 bis 100 Gew.-% und ganz besonders bevorzugt solches mit einer Reinheit von 97 bis 99,9 Gew.-% oder noch mehr bevorzugt 98 bis 99,8 Gew.-%.

Ein weiterer Ausgangsstoff für das erfindungsgemäße Verfahren ist ein Aldehyd der Formel (IV) R¹-CHO, wobei R¹ in der Formel (IV) die zuvor angegebene Bedeutung hat.

Bevorzugt steht R¹ in den Verbindungen der Formeln (I), (II) und (IV) für geradkettiges oder verzweigtes C₁-C₁₂-Alkyl oder geradkettiges oder verzweigtes C₂-C₁₂-Alkenyl. Besonders bevorzugt steht R¹ für geradkettiges oder verzweigtes C₁-C₆-Alkyl oder geradkettiges oder verzweigtes C₂-C₆-Alkenyl. In einer weiteren bevorzugten Ausführung steht R¹ für Phenyl.

Erfindungsgemäß bevorzugte Bedeutungen für den Rest R¹ sind somit beispielsweise Methyl, Ethyl, n-Propyl, Isopropyl, n-Butyl, Isobutyl, n-Pentyl, n-Hexyl oder n-Heptyl, bevorzugt Methyl, Ethyl, n-Propyl, Isopropyl, n-Butyl, Isobutyl, ganz besonders bevorzugt Isobutyl (2-Methylpropyl).

Besonders bevorzugt steht der Rest R¹ für Isobutyl oder Phenyl.

Bevorzugt einzusetzende Aldehyde der Formel (IV) sind: Acetaldehyd, Valeraldehyd, Isovaleraldehyd, Pentanal, Hexanal, Heptanal, Benzaldehyd, Citral, Citronellal. Erfindungsgemäß ganz besonders bevorzugt einzusetzende Aldehyde der Formel (IV) sind Isovaleraldehyd und Benzaldehyd, insbesondere Isovaleraldehyd.

Die Umsetzung der Verbindungen (III) und (IV) erfolgt in einer Anordnung aus n in Reihe geschalteter Reaktoren. Dabei steht n für eine natürliche Zahl von wenigstens zwei. Erfindungsgemäß handelt es sich um 2 bis 8, vorzugsweise 2, 3, 4, 5 oder 6 in Strömungsrichtung hintereinander angeordnete Reaktoren.

In einer erfindungsgemäßen Anordnung können auch ein einzelner, mehrere oder alle der in Reihe geschalteten Reaktoren durch zwei oder mehr parallel geschaltete Reaktoren ersetzt werden. Daraus kann sich eine kombinierte Reihen- und Parallelschaltung von (n+m) Reaktoren ergeben. Die Anzahl der Reaktoren in der längsten Reihe hintereinander geschalteter Reaktoren ergibt n. Die Anzahl aller anderen Reaktoren ergibt in Summe m, wobei m eine beliebige natürliche Zahl sein kann.

Vorzugsweise erfolgt die Umsetzung kontinuierlich. Das bedeutet, dass alle n in Reihe geschalteten Reaktoren jeweils kontinuierlich betrieben werden.

In einer geeigneten Ausführungsform erfolgt die Umsetzung in Gegenwart eines Lösungsmittels. Gegebenenfalls werden zur Durchführung der erfindungsgemäßen Umsetzung die Verbindungen der Formeln (III) und (IV), hier und im Folgenden auch als Ausgangsstoffe oder Edukte bezeichnet, jeweils in Form eines Gemisches mit einem geeigneten Lösungsmittel zugeführt. Bevorzugt werden beide Ausgangsstoffe (III) und (IV) im gleichen Lösungsmittel vorgelegt. Bei dem Lösungsmittel handelt es sich vorzugsweise um Wasser oder ein unter den Reaktionsbedingungen inertes Lösungsmittel wie beispielsweise tert-Butylmethylether, Cyclohexan, Toluol, Hexan oder Xylol. Die genannten Lösungsmittel können alleine oder in Form von Gemischen eingesetzt werden. In einer bevorzugten Ausgestaltung führt man die Umsetzung ohne Zusatz eines organischen Lösungsmittels durch. In einer besonders bevorzugten Ausgestaltung erfolgt die Umsetzung in Gegenwart von Wasser.

In dem erfindungsgemäßen Verfahren wird zwischen dem in Strömungsrichtung ersten und letzten Reaktor einen Teilstrom entnommen und in einen stromaufwärts der Entnahmestelle gelegenen Reaktor eingespeist.

In einer bevorzugten Ausgestaltung wird dem Reaktoraustrag des in Strömungsrichtung ersten und/oder zweiten Reaktors ein Teilstrom entnommen und über eine externe Rückführung zumindest teilweise in den in Strömungsrichtung ersten Reaktor zurückgeführt. Gemäß dieser Ausgestaltung wird zumindest der in Strömungsrichtung erste Reaktor rückvermischt betrieben.

Insbesondere wird ein Teilstrom des Reaktoraustrags aus dem ersten Reaktor abgezogen und über einen externen Kreislauf in den in Strömungsrichtung ersten Reaktor zurückgeführt. Diese Betriebsweise wird hier und im Folgenden auch als Schlaufenfahrweise bezeichnet. Vorzugsweise steht n dabei für zwei. Die Stromteilung für die Rückführung kann gegebenenfalls vor oder nach einer Zwischenkühlung erfolgen.

Gemäß einer alternativen Ausgestaltung erfolgt die Umsetzung in n hintereinander geschalteten Reaktoren, wobei n für eine ganze Zahl von wenigstens drei steht. In dieser Ausgestaltung wird ein Teilstrom aus dem (n-1)-ten Reaktor abgezogen und über einen externen Kreislauf mit dem in den ersten Reaktor zugeführten Strom zurückgeführt. Damit bilden der erste bis (n-1)-te Reaktor gemeinsam eine Schlaufe. Insbesondere steht n dabei für drei. Auch hier kann die Stromteilung für die Rückführung gegebenenfalls vor oder nach einer Zwischenkühlung erfolgen.

In einer besonders bevorzugten Ausgestaltung wird dem Teilstrom vor der Einspeisung in einen stromaufwärts der Entnahmestelle gelegenen Reaktor Wärme entzogen.

In einer Ausführungsform des erfindungsgemäßen Verfahrens wird zumindest der in Strömungsrichtung erste Reaktor weitgehend isotherm betrieben.

Unter "weitgehend isotherm betrieben" soll im Rahmen der vorliegenden Erfindung verstanden werden, dass in der jeweiligen Reaktionszone ein enges Temperaturintervall eingehalten wird. Wird der Reaktor "weitgehend isotherm betrieben", so soll im Rahmen der vorliegenden Erfindung darunter verstanden werden, dass das Temperaturintervall ΔT im Reaktor kleiner als die adiabatische Temperaturerhöhung ist. Für das Temperaturintervall in einem Reaktor gilt bevorzugt ΔT ≤12 K, besonders bevorzugt Δ T ≤ 10 K.

Für eine weitgehend isotherme Fahrweise werden geeigneter Weise Wärmeübertragungsflächen im Inneren des ersten Reaktors angeordnet. In diesem Fall kann auf eine Rückvermischung im ersten Reaktor verzichtet werden. Wird ein Reaktor "im geraden Durchgang" betrieben, so soll hier und im Folgenden darunter verstanden werden, dass ein Reaktor ohne Rückführung des Reaktionsproduktes im Sinne der Schlaufenfahrweise betrieben wird. Die Betriebsweise im geraden Durchgang schließt dabei rückvermischende Einbauten und/oder Rühreinrichtungen im Reaktor grundsätzlich nicht aus.

In einer geeigneten Ausgestaltung werden der in Strömungsrichtung erste und zweite Reaktor jeweils weitgehend isotherm betrieben. Dazu werden geeigneter Weise Wärmeübertragungsflächen im Inneren der beiden ersten Reaktoren angeordnet. Auf diese Weise lassen sich gegebenenfalls unterschiedliche Temperaturniveaus in den Reaktoren einstellen. In diesem Fall kann auf eine Rückvermischung im ersten und im zweiten Reaktor verzichtet werden.

In einer geeigneten Ausführungsform werden ein, mehrere oder alle in einen Reaktor zugeführten Ströme jeweils vor Eintritt in den Reaktor temperiert. Dazu kann ein üblicher Wärmeübertrager eingesetzt werden. In der Regel wird der aus einem Reaktor abgezogene Strom vor Eintritt in den nachfolgenden Reaktor zwischengekühlt. Mit der dabei gewonnenen Wärme ist es möglich, einen Strom an einer geeigneten anderen Stelle des Verfahrens zu erwärmen. Dem Fachmann sind entsprechende Verfahren zur Wärmeintegration bzw. Pinch-Analyse bekannt.

In einer besonders vorteilhaften Ausführungsform des erfindungsgemäßen Verfahrens wird dem Reaktoraustrag aus mindestens einem der ersten bis (n-1)-ten Reaktoren vor Zuführung in den in Strömungsrichtung folgenden Reaktor Wärme entzogen.

In einer ebenfalls bevorzugten Ausführungsform wird zumindest der in Strömungsrichtung letzte Reaktor ohne Rückführung des Reaktoraustrags betrieben. Eine vollständige oder teilweise Produktrückführung nach Austritt aus dem in Strömungsrichtung letzten Reaktor ist im kontinuierlichen Betrieb vorzugsweise nicht vorzusehen.

Insbesondere wird der in Strömungsrichtung letzte Reaktor im Wesentlichen ohne Rückvermischung betrieben. In diesem Fall ist als in Strömungsrichtung letzter Reaktor speziell ein Rohrreaktor ohne rückvermischende Einbauten vorgesehen.

Bevorzugt steht n für 2 oder 3. Besonders bevorzugt steht n für 2.

In einer geeigneten Ausführungsform des erfindungsgemäßen Verfahrens wird die Umsetzung zumindest in dem in Strömungsrichtung letzten Reaktor adiabatisch durchgeführt.

Der Begriff "adiabatisch" wird im Rahmen der vorliegenden Erfindung im technischen und nicht im physikalisch-chemischen Sinne verstanden. So erfährt das Reaktionsgemisch beim Strömen durch den Reaktor auf Grund der exothermen Reaktion in der Regel eine Temperaturerhöhung. Unter adiabatischer Reaktionsführung wird eine Vorgehensweise verstanden, bei der die bei der Reaktion freiwerdende Wärmemenge von der Reaktionsmischung im Reaktor aufgenommen und keine Kühlung durch Kühlvorrichtungen erfolgt. Somit wird die Reaktionswärme weitestgehend mit dem Reaktionsgemisch aus dem Reaktor abgeführt. Es liegt auf der Hand, dass ein Restanteil durch natürliche Wärmeleitung bzw. -strahlung vom Reaktor an die Umgebung abgegeben wird. Vorzugsweise wird der letzte Reaktor dabei im geraden Durchgang betrieben.

Gemäß einer bevorzugten Ausführungsform wird zur Umsetzung eine Reaktoranordnung verwendet, die wenigstens einen Festbettreaktor umfasst. Besonders bevorzugt wird eine Reaktoranordnung eingesetzt, in der alle n Reaktoren Festbettreaktoren sind.

Gemäß einer geeigneten Ausführungsform wird zur Umsetzung eine Reaktoranordnung verwendet, die wenigstens einen Reaktor mit einem intern angeordneten Wärmeübertrager umfasst.

Vorzugsweise erfolgt die Umsetzung in Gegenwart eines sauren Katalysators, der ausgewählt ist unter Salzsäure, Schwefelsäure, Methansulfonsäure, p-Toluolsulfonsäure und stark sauren Kationenaustauschern. Insbesondere wird die Umsetzung in Gegenwart eines stark sauren Kationenaustauschers durchgeführt.

Bevorzugt werden der Alkohol der Formel (III) und der Aldehyd der Formel (IV) in einem molaren Verhältnis im Bereich von 0,7 : 1 bis 2 : 1 eingesetzt.

Vorzugsweise werden der Alkohol der Formel (III) und der Aldehyd der Formel (IV) in Gegenwart von mindestens 3 Gew.-%, besonders bevorzugt von mindestens 5 Gew.-% Wasser umgesetzt. Der Alkohol der Formel (III) und der Aldehyd der Formel (IV) werden beispielsweise in Gegenwart von 3 Gew.-% bis 15 Gew.-% Wasser, bevorzugt von 5 Gew.-% bis 12 Gew.-% umgesetzt. Die angegebenen vorstehenden Gew.-% sind dabei bezogen auf die Menge des Reaktionsgemisches, bestehend aus den Komponenten der Formeln (III) und (IV)sowie Wasser.

In der Regel führt man die Umsetzung des Alkohols der Formel (III) mit dem Aldehyd der Formel (IV) in Gegenwart von etwa mindestens 10 Mol-% Wasser durch, wobei sich die Menge an Wasser auf die Menge des gegebenenfalls im Unterschuss eingesetzten Ausgangsstoffes, oder im Fall einer äquimolaren Umsetzung auf die Stoffmenge eines der beiden Ausgangsstoffe bezieht. Oberhalb des angegebenen Wertes kann die Menge an Wasser frei gewählt werden und ist nur durch verfahrenstechnische oder ökonomische Aspekte limitiert. Wasser kann auch in großem, beispielsweise in 10- bis 100-fachem Überschuss oder sogar darüber eingesetzt werden. Vorzugsweise bereitet man ein Gemisch aus dem Alkohol der Formel (III) und dem Aldehyd der Formel (IV) mit der gewählten Menge Wasser, so dass das zugegebene Wasser in dem Gemisch gelöst bleibt, d. h. dass kein zweiphasiges System vorliegt.

In einer geeigneten Ausgestaltung werden die Ausgangsstoffe in Gegenwart von mindestens 25 Mol-%, bevorzugt von mindestens 50 Mol-% Wasser umgesetzt. Beispielsweise werden die Ausgangsstoffe in Gegenwart von 25 bis 150 Mol-%, bevorzugt von 40 bis 150 Mol-%, besonders bevorzugt von 50 bis 140 Mol-%, insbesondere von 50 bis 80 Mol-% Wasser umgesetzt. Dabei bezieht sich die Menge an eingesetztem Wasser auf die Stoffmenge des gegebenenfalls im Unterschuss eingesetzten Ausgangsstoffes oder im Fall einer äquimolaren Umsetzung auf die Stoffmenge eines der beiden.

In einer geeigneten Ausführungsform des erfindungsgemäßen Verfahrens wird die Umsetzung bei einer Temperatur im Bereich von 0 °C bis 70 °C, bevorzugt im Bereich von 20 °C bis 70 °C, besonders bevorzugt im Bereich von 20 °C bis 60 °C durchgeführt.

In einer ebenfalls geeigneten Ausführungsform des erfindungsgemäßen Verfahrens wird die Umsetzung bei einem Druck im Bereich von 1 bar bis 15 bar durchgeführt.

Sofern das in einem der dem ersten Reaktor nachgeschalteten Reaktoren (d. h. dem zweiten bis n-ten Reaktor) umgesetzte Reaktionsgemisch zu geringe Anteile an Edukten aufweist, um über die auftretende Reaktionswärme die gewünschte Temperatur im Reaktor zu halten, kann auch eine Temperierung des Reaktors (oder einzelner Reaktionszonen) erforderlich sein. Die Temperierung kann analog der zuvor beschriebenen Abfuhr der Reaktionswärme durch Erwärmung eines externen Umlaufstroms oder durch interne Erwärmung über Wärmeübertragungsflächen erfolgen. In einer geeigneten Ausführung kann zur Temperierung die abgeführte Reaktionswärme aus wenigstens einem der vorherigen Reaktoren verwendet werden.

Die abgezogene Reaktionswärme kann gegebenenfalls auch zur Erwärmung der Feedströme der Reaktoren verwendet werden. Dazu kann z. B. der Eduktstrom in den ersten Reaktor zumindest teilweise mit einem externen Umlaufstrom dieses Reaktors gemischt und die vereinigten Ströme dann in den ersten Reaktor geführt werden. Des Weiteren können die Feedströme in einen, mehrere oder alle der zweiten bis n-ten Reaktoren mit einem Umlaufstrom aus dem jeweiligen Reaktor gemeinsam in diesen Reaktor geführt werden. Des Weiteren kann der Eduktstrom und/oder ein anderer Feedstrom mit Hilfe eines Wärmetauschers erwärmt werden, der mit entzogener Reaktionswärme betrieben wird.

In einer Ausführungsform kann in wenigstens einem der eingesetzten Reaktoren eine zusätzliche Durchmischung erfolgen. Eine zusätzliche Durchmischung ist insbesondere vorteilhaft, wenn die Reaktion bei großen Verweilzeiten des Reaktionsgemischs erfolgt. Geeignet sind sowohl statische als auch dynamische Mischvorrichtungen. Geeignete Mischvorrichtungen sind dem Fachmann hinlänglich bekannt. Zur Durchmischung können bevorzugt die in die Reaktoren eingespeisten Feedströme über geeignete Mischvorrichtungen, wie Düsen, in die jeweiligen Reaktoren eingespeist werden. Zur Durchmischung können ebenfalls bevorzugt in einem externen Kreislauf geführte (Teil-) Ströme aus dem jeweiligen Reaktor eingesetzt werden, wie oben als Schlaufenfahrweise beschrieben.

Die zuvor beschriebene Schlaufenfahrweise eignet sich besonders vorteilhaft zur Regulierung der Reaktionstemperatur und des Wärmeübergangs zwischen Reaktionsmedium, Apparatewänden und Umgebung. Eine weitere Möglichkeit zur Steuerung der Wärmebilanz besteht in der Regelung der Eintrittstemperatur des Eduktes bzw. des jeweiligen Feedstromes. So führt eine tiefere Temperatur des eintretenden Feeds in der Regel zu einer verbesserten Abführung der Reaktionswärme. Beim Nachlassen der Katalysatoraktivität kann die Eintrittstemperatur höher gewählt werden, um eine höhere Reaktionsgeschwindigkeit zu erreichen und somit die nachlassende Katalysatoraktivität zu kompensieren. So kann die Standzeit des eingesetzten Katalysators in vorteilhafter Weise erhöht werden.

Der erste Teilstrom wird im Allgemeinen chemisch unverändert in das Reaktionssystem zurückgeführt. Sofern gewünscht, können die Temperatur und/oder der Druck vor der Zurückführung auf die gewünschten Werte eingestellt werden. Die Einspeisung des ersten Teilstroms in den Reaktor, dem er entnommen wurde, kann gemeinsam mit dem jeweiligen Feedstrom oder separat davon erfolgen. Das Gewichtsmengenverhältnis von in einen Reaktor eingespeistem ersten Teilstrom (Rückführstrom) zu jeweiligem Feedstrom liegt vorzugsweise in einem Bereich von 1 : 1 bis 50 : 1, besonders bevorzugt in einem Bereich von 2 : 1 bis 30 : 1, insbesondere im Bereich von 5 : 1 bis 20 : 1.

In einer zweiten Variante erfolgt die Umsetzung in Gegenwart eines stark sauren Kationenaustauschers. Unter dem Begriff stark saurer Kationenaustauscher wird dabei ein Kationenaustauscher in der H⁺-Form verstanden, der stark saure Gruppen aufweist. Bei den stark sauren Gruppen handelt es sich in der Regel um Sulfonsäuregruppen. Die sauren Gruppen sind in der Regel angebunden an eine Polymermatrix, die z. B. gelförmig bzw. makroporös sein kann. Eine bevorzugte Ausführungsform des erfindungsgemäßen Verfahrens ist dementsprechend dadurch gekennzeichnet, dass man einen stark sauren, Sulfonsäuregruppen aufweisenden Kationenaustauscher einsetzt. Geeignete stark saure Kationenaustauscher sind in der WO 2010/133473 und WO 2011/154330 beschrieben.

Geeignet für den Einsatz sind stark saure Ionenaustauscher (wie z. B. Amberlyst, Amberlite, Dowex, Lewatit, Purolite, Serdolit), die auf Polystyrol basieren und die Copolymere aus Styrol und Divinylbenzol als Trägermatrix mit Sulfonsäuregruppen in H⁺-Form enthalten sowie mit Sulfonsäuregruppen (-SO₃H) funktionalisierte Ionenaustauschergruppen. Die Ionenaustauscher unterscheiden sich im Aufbau ihrer Polymergerüste, und man unterscheidet gelförmige und makroporöse Harze. In einer speziellen Ausführung wird ein perfluoriertes polymeres Ionenaustauscherharz eingesetzt. Derartige Harze werden z. B. unter der Bezeichnung Nation ® von der Firma DuPont vertrieben. Als Beispiel für ein solches perfluoriertes polymeres Ionenaustauscherharz sei Nation ® NR-50 genannt.

Für die Umsetzung geeignete kommerziell verfügbare stark saure Kationenaustauscher sind beispielsweise unter den Handelsnamen Lewatit ® (Lanxess), Purolite ® (The Purolite Company), Dowex ® (Dow Chemical Company), Amberlite ® (Rohm and Haas Company), Amberlyst (TM) (Rohm and Haas Company) bekannt. Bevorzugte stark saure Kationenaustauscher sind: Lewatit ® K 1221, Lewatit ® K 1461, Lewatit ® K 2431, Lewatit ® K 2620, Lewatit ® K 2621, Lewatit ® K 2629, Lewatit ® K 2649, Amberlite ® FPC 22, Amberlite ® FPC 23, Amberlite ® IR 120, Amberlyst (TM) 131, Amberlyst (TM) 15, Amberlyst (TM) 31, Amberlyst (TM) 35, Amberlyst (TM) 36, Amberlyst (TM) 39, Amberlyst (TM) 46, Amberlyst (TM) 70, Purolite ® SGC650, Purolite ® C100H, Purolite ® C150H, Dowex ® 50X8, Serdolit ® rot und Nation ® NR-50.

Die stark sauren lonentauscherharze werden in der Regel mit Salzsäure und/oder Schwefelsäure regeneriert.

In einer speziellen Ausführung werden das 3-Methylbut-3-en-ol (III) und der Aldehyd (IV) in Gegenwart eines stark sauren Kationenaustauschers und in Gegenwart von Wasser umgesetzt. Prinzipiell kann das Reaktionsgemisch bereits geringe Mengen Wasser enthalten, das durch die Dehydratisierung des Verfahrensproduktes der Formel (I) als mögliche Nebenreaktion freigesetzt werden kann. Nach einer speziellen Ausführung wird dem Reaktionsgemisch neben Isoprenol (III) und dem Aldehyd der Formel (IV) sowie etwaigem Wasser aus der Reaktion zusätzlich noch Wasser zugesetzt.

Vorzugsweise werden der Alkohol der Formel (III) und der Aldehyd der Formel (IV) in Gegenwart von mindestens 3 Gew.-%, besonders bevorzugt von mindestens 5 Gew.-% Wasser umgesetzt. Der Alkohol der Formel (III) und der Aldehyd der Formel (IV) werden beispielsweise in Gegenwart von 3 Gew.-% bis 15 Gew.-% Wasser, bevorzugt von 5 Gew.-% bis 12 Gew.-% umgesetzt. Die angegebenen vorstehenden Gew.-% sind dabei bezogen auf die Gesamtmenge des Reaktionsgemisches, bestehend aus den Komponenten der Formeln (III) und (IV) sowie Wasser.

Oberhalb des angegebenen Wertes kann die Menge an Wasser frei gewählt werden und ist, wenn überhaupt, nur durch verfahrenstechnische oder ökonomische Aspekte begrenzt und kann durchaus in großem, beispielsweise in 5- bis 15-fachem Überschuss oder auch darüber eingesetzt werden. Vorzugsweise bereitet man ein Gemisch aus Isoprenol (III) und dem Aldehyd der Formel (IV), vorzugsweise Isovaleraldehyd, mit der zuzusetzenden Menge Wasser, so dass das zugegebene Wasser in dem Gemisch aus Isoprenol und dem Aldehyd gelöst bleibt d. h. kein zweiphasiges System vorliegt.

Üblicherweise setzt man im Rahmen dieser Ausführungsform des erfindungsgemäßen Verfahrens die Ausgangsstoffe Isoprenol (III) und den Aldehyd der Formel (IV) in Gegenwart von mindestens 25 mol-%, bevorzugt von mindestens 50 mol-% um. Beispielsweise werden die Ausgangsstoffe in Gegenwart von 25 bis 150 Mol-%, bevorzugt von 40 bis 150 Mol-%, besonders bevorzugt von 50 bis 140 Mol-%, insbesondere von 50 bis 80 Mol-% Wasser umgesetzt. Dabei bezieht sich die Menge an eingesetztem Wasser auf die Stoffmenge des gegebenenfalls im Unterschuss eingesetzten Ausgangsstoffes oder im Fall einer äquimolaren Umsetzung auf die Stoffmenge eines der beiden.

Zur Umsetzung von Isoprenol (III) mit dem Aldehyd (IV) kann man die genannten Ausgangsstoffe und gegebenenfalls das zugesetzte Wasser mit dem sauren Kationenaustauscher in Kontakt bringen. Vorzugsweise werden Isoprenol (III), Aldehyd (IV) und gegebenenfalls das zugesetzte Wasser in Form eines Gemisches eingesetzt. Die genannten Ausgangsstoffe, d. h. Isoprenol (III) und der Aldehyd (IV) und das in der vorstehenden Menge einzusetzende Wasser können in beliebiger Reihenfolge miteinander in Kontakt gebracht bzw. gemischt werden.

Die Menge an stark saurem Kationenaustauscher ist nicht kritisch und kann unter Berücksichtigung des wirtschaftlichen und verfahrenstechnischen Aspektes in breiten Grenzen frei gewählt werden. Die Umsetzung kann dementsprechend sowohl in Gegenwart katalytischer Mengen als auch in Gegenwart großer Überschüsse des stark sauren Kationenaustauschers durchgeführt werden. Die genannten stark sauren Kationenaustauscher können sowohl einzeln oder auch in Form von Gemischen eingesetzt werden.

Die Katalysatorbelastung liegt beispielsweise im Bereich von 50 bis 2500 mol pro m³ Katalysator und h, bevorzugt im Bereich von 100 bis 2000 mol pro m³ Katalysator und h, insbesondere im Bereich von 130 bis 1700 mol pro m³ Katalysator und h, wobei sich die Stoffmenge in mol auf den Ausgangsstoff der Formel (IV) bezieht.

Die Umsetzung in Gegenwart eines stark sauren Kationenaustauschers kann wahlweise auch zusätzlich in Gegenwart eines unter den Reaktionsbedingungen inerten Lösungsmittels durchgeführt werden. Geeignete Lösungsmittel sind beispielsweise tert-Butylmethylether, Cyclohexan, Dekalin, Hexan, Heptan, Ligroin, Petrolether, Toluol oder Xylol. Die genannten Lösungsmittel können alleine oder in Form von Gemischen untereinander eingesetzt werden. Bevorzugt führt man die Umsetzung in Gegenwart eines stark sauren Kationenaustauschers ohne Zusatz eines organischen Lösungsmittels durch.

Bevorzugt wird die Umsetzung von Isoprenol (III) mit dem gewählten Aldehyd (IV) in Gegenwart von Wasser und in Gegenwart eines stark sauren Kationentauschers bei einer Temperatur im Bereich von 0 bis 70 °C, besonders bevorzugt bei einer Temperatur im Bereich von 20 bis 70 °C und insbesondere bei einer Temperatur im Bereich von 20 bis 60 °C durchgeführt. Dabei handelt es sich um die Temperatur des Reaktionsgemisches.

Die Aufarbeitung des Reaktionsaustrags zur Gewinnung des Wertprodukts kann nach üblichen, dem Fachmann bekannten Verfahren erfolgen. Bevorzugt umfasst die Aufarbeitung des Reaktionsgemisches wenigstens einen Destillationsschritt. Der Reaktionsaustrag kann sich in bekannter Weise durch Destillation bzw. Rektifikation aufgetrennt werden, um so das Wertprodukt zu erhalten. Beispielsweise kann die Aufarbeitung analog zu der in der WO 2011/154330 beschriebenen Methode erfolgen.

### FIGURENBESCHREIBUNG

Das erfindungsgemäße Verfahren wird anhand der Figuren 1 bis 3 im Folgenden näher erläutert, ohne es auf diese Ausführungsformen einzuschränken.
- Figur 1: zeigt eine Ausführungsform des erfindungsgemäßen Verfahrens mit einem Hauptreaktor mit Rückführstrom und einem Nachreaktor.
- Figur 2: zeigt eine nicht erfindungsgemäße Ausführungsform des erfindungsgemäßen Verfahrens mit einem Hauptreaktor mit integriertem Wärmeaustauscher und einem Nachreaktor.
- Figur 3: zeigt eine Ausführungsform des erfindungsgemäßen Verfahrens mit zwei Reaktorstufen mit Rückführstrom und einem Nachreaktor.

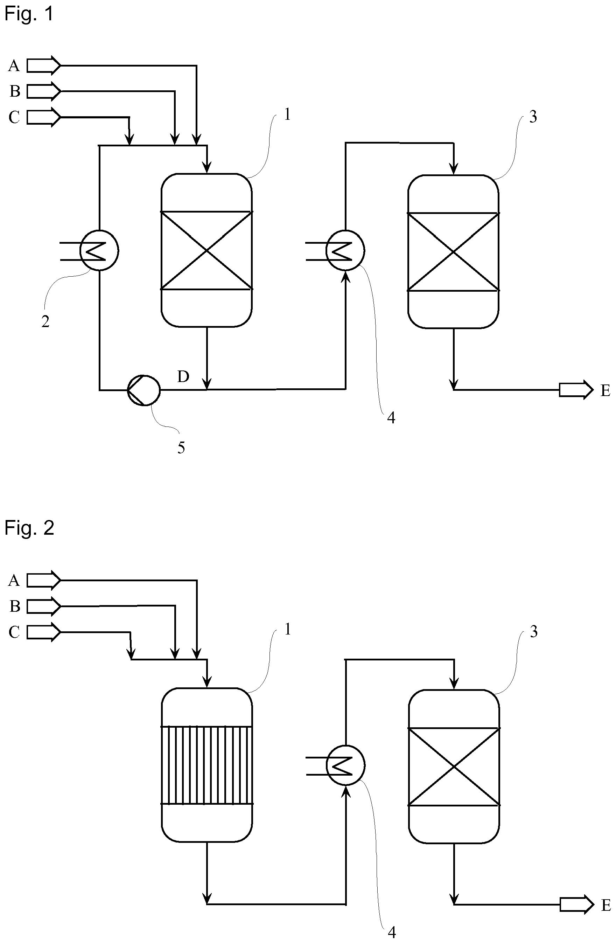

In den Figuren 1 bis 3 werden folgende Bezugszeichen verwendet:
- 1: (Haupt-)Reaktor
- 2: Kühler
- 3: (Nach-)Reaktor
- 4: (Zwischen-)Kühler
- 5: Pumpe
- 6: Reaktor
- 7: Kühler
- 8: Trennkolonne

- A: Isoprenol-Strom
- B: Aldehyd-Strom
- C: Wasser
- D: Rückführstrom
- E: Edukt

Das erfindungsgemäße Verfahren kann mit mindestens einem Hauptreaktor, bevorzugt 1 bis 2 Hauptreaktoren, kaskadiert durchgeführt werden. Die Hauptreaktoren können parallel oder in Reihe, bevorzugt in Reihe, und gegebenenfalls mit Zwischenkühlung betrieben werden. Dabei kann beispielsweise im rückvermischten Reaktorsystem oder in isothermer Fahrweise gefahren werden. Im rückvermischten Reaktorsystem kann der gesamte Umlaufstrom des Hauptreaktorteils rückvermischt und gekühlt oder jeder Hauptreaktor für sich durch einen eigenen Umlaufstrom rückvermischt und gekühlt werden und/oder nach jedem Hauptreaktor zwischengekühlt werden kann. Die Aufteilung in mehrere Betten, gegebenenfalls auch mit Zwischenkühlung, kann auch in einem Apparat umgesetzt werden.

Nach dem Austritt aus dem Hauptreaktorteil der Umsetzung folgt mindestens ein Nachreaktor, bevorzugt 1 bis 2 Nachreaktoren. Diese können im geraden Durchlauf (isotherm oder rückvermischt), parallel oder in Reihe betrieben werden. Bevorzugt werden sie in Reihe geschaltet und in geradem Durchlauf ohne Rückvermischung betrieben.

Figur 1 zeigt eine geeignete Ausführungsform einer geeigneten zweistufigen Reaktorkaskade mit einem Hauptreaktor (1) und einem Nachreaktor (3).

Über drei Zuführungen werden die drei Eduktströme Isoprenol (A), Aldehyd (B) und Wasser (C) in den Reaktor (1) eingeleitet. Über eine Leitung und die Pumpe (5) wird ein Austrag aus dem Reaktor (1) entnommen, der in zwei Teilströme geteilt wird. Über den Kühler (2) wird ein Rückführstrom (D) mit den Eduktströmen (A), (B) und (C) zusammen in den Hauptreaktor (1) geführt. Ein Feedstrom wird über einen Kühler (4) dem zweiten Reaktor (3) zugeführt. Das Edukt (E) wird als Austrag aus dem Nachreaktor (3) direkt entnommen und gegebenenfalls einer Aufarbeitung zugeführt.

Beide Reaktoren werden in dieser Ausgestaltung bevorzugt als Festbettreaktoren ausgeführt. Der Hauptreaktor (1) wird in Schlaufenfahrweise betrieben, wohingegen der Nachreaktor im geraden Durchgang betrieben wird. In der in Figur 1 dargestellten Anordnung sind der Hauptreaktor (1) und der Nachreaktor (3) so in Reihe geschaltet, dass über eine Rückvermischung im Hauptreaktorsystem die Einstellung eines Temperaturprofils über dem Katalysatorbett erfolgen kann. Dadurch kann ein starker Temperaturanstieg zu Beginn der Reaktion verhindert werden.

In Figur 2 wird eine nicht erfindungsgemäße Ausführungsform einer zweistufigen Reaktorkaskade mit einem Hauptreaktor (1) und einem Nachreaktor (3). gezeigt. Statt der Rückführung wird über einen in den Reaktor (1) integrierten Wärmeübertrager eine isotherme Reaktionsführung erzielt.

Über drei Zuführungen werden die drei Eduktströme Isoprenol (A), Aldehyd (B) und Wasser (C) in den Reaktor (1) eingeleitet. Aus dem Reaktor (1) wird ein Austrag entnommen, der als Feedstrom über einen Kühler (4) dem zweiten Reaktor (3) zugeführt wird. Das Edukt (E) wird als Austrag aus dem Nachreaktor (3) direkt entnommen und gegebenenfalls einer Aufarbeitung zugeführt. Der Hauptreaktor ist mit integrierten Wärmeübertragungsflächen ausgestattet, wohingegen der Nachreaktor (3) als einfacher Festbettreaktor ausgeführt ist. Beide Reaktoren werden in dieser Ausgestaltung geraden Durchgang betrieben. Durch die in Figur 2 veranschaulichte isotherme Reaktionsführung werden unerwünschte Temperaturspitzen vermieden.

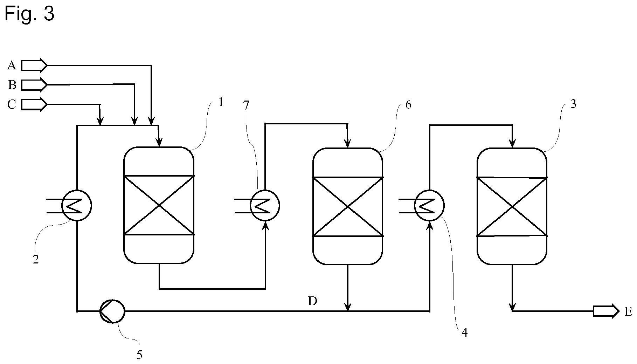

Figur 3 zeigt eine geeignete Ausführungsform einer dreistufigen Reaktorkaskade mit zwei Hauptreaktoren (1), (6) und einem Nachreaktor (3).

Über drei Zuführungen werden die drei Eduktströme Isoprenol (A), Aldehyd (B) und Wasser (C) in den Reaktor (1) eingeleitet. Aus dem Reaktor (1) wird ein Austrag entnommen, der als Feedstrom über einen Kühler (7) dem zweiten Reaktor (6) zugeführt wird. Über eine Leitung und die Pumpe (5) wird ein Austrag aus dem Reaktor (6) entnommen, der in zwei Teilströme geteilt wird. Über den Kühler (2) wird ein Rückführstrom (D) mit den Eduktströmen (A), (B) und (C) zusammen in den Hauptreaktor (1) zurückgeführt. Ein Feedstrom wird über einen Kühler (4) dem dritten Reaktor (3) zugeführt. Das Edukt (E) wird als Austrag aus dem Nachreaktor (3) direkt entnommen und gegebenenfalls einer Aufarbeitung zugeführt.

Alle drei Reaktoren werden in dieser Ausgestaltung bevorzugt als Festbettreaktoren ausgeführt. Die Hauptreaktoren (1) und (6) werden gemeinsam in Schlaufenfahrweise betrieben, wohingegen der Nachreaktor (3) im geraden Durchgang betrieben wird. In der in Figur 3 dargestellten Anordnung sind die Hauptreaktoren (1), (6) und der Nachreaktor (3) so in Reihe geschaltet, dass über eine Rückvermischung im Hauptreaktorsystem und eine Zwischenkühlung zwischen dem ersten und zweiten Hauptreaktor die Einstellung eines Temperaturprofils über dem Katalysatorbett erfolgen kann. Dadurch können Temperaturspitzen in beiden Reaktoren wirksam verhindert werden.

### BEISPIELE

### Beispiel 1 (kontinuierlich betriebener Prozess)

Es wurde eine Apparatur aus einem Hauptreaktor und einem aus drei Einzelreaktoren bestehenden Nachreaktor verwendet. Als Hauptreaktor wurde ein Doppelmantelreaktor aus RA4 ohne Heizmedium für eine adiabatische Fahrweise mit einer Länge von 150 cm und einem Innendurchmesser von 2,6 cm verwendet. Als Nachreaktor wurden drei Doppelmantelreaktoren aus RA4 mit einer Länge von je 150 cm, mit einem Innendurchmesser von je 1,0 cm und beheizt mit jeweils 30 °C, 40 °C sowie 50 °C, eingesetzt.

Die Apparatur wurde mit insgesamt 328 g des stark sauren Kationenaustauschers Amberlyst™ 131 gefüllt. Der Hauptreaktor wurde dabei mit 230 g (305 ml), die Nachreaktoren mit je 32,5 g (44 ml) des Kationenaustauschers gefüllt. Der Kationenaustauscher wurde vor dem Einsatz zunächst mehrmals mit Wasser, dann einmal mit Methanol und abschließend mit Wasser Methanol-frei gewaschen. Das System wurde durch Zufahren einer Mischung aus Pyranol : Wasser im Massen-Verhältnis von 95 : 5 konditioniert. Der Hauptreaktor wurde anschließend rückvermischt mit einem Umwälzstrom von 2000 g/h betrieben, wobei der rückgeführte Strom vor Wiedereintritt in den Hauptreaktor auf eine Temperatur von 25 °C abgekühlt wurde. Der Nachreaktor wurde im geraden Durchgang zum Vollumsatz betrieben.

Nach Konditionierung des Kationenaustauschers auf das genannte Pyranol-WasserGemisch wurde ein Gemisch aus Isovaleraldehyd : Isoprenol : Wasser im Massen-Verhältnis von 45 : 50 : 5 bei 25 °C und mit einem Gesamtmengenstrom von 100 g/h zugefahren. Man erhielt ein Rohprodukt mit einer Austrittstemperatur aus dem letzten Nachreaktor von 50 °C in einer Ausbeute von 76 % und mit einer Selektivität von 77,6 %, jeweils bezogen auf Isovaleraldehyd mit der folgenden Zusammensetzung:
Isovaleraldehyd: 1,03 GC-Gew.-%,
Isoprenol: 3,6 GC-Gew.-%,
Dihydropyran-Isomere: 8,69 GC-Gew.-%,
1,3-Dioxan: 5,56 GC-Gew.-%,
Acetal: 0,57 GC-Gew.-%,
trans-Pyranol: 18,26 GC-Gew.-%,
cis-Pyranol: 50,08 GC-Gew.-%,
Wasser: 6,8 Gew.-% (nach Karl Fischer).

## Patentansprüche

1. Verfahren zur Herstellung von 2-substituierten 4-Hydroxy-4-methyl-tetrahydropyranen der allgemeinen Formel (I) 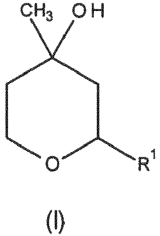 worin
R¹ für geradkettiges oder verzweigtes C₁-C₁₂-Alkyl, geradkettiges oder verzweigtes C₂-C₁₂-Alkenyl, unsubstituiertes oder mit C₁-C₁₂-Alkyl und/oder C₁-C₁₂-Alkoxy substituiertes Cycloalkyl mit insgesamt 3 bis 20 Kohlenstoffatomen oder unsubstituiertes oder mit C₁-C₁₂-Alkyl und/oder C₁-C₁₂-Alkoxy substituiertes Aryl mit insgesamt 6 bis 20 Kohlenstoffatomen steht,
umfassend eine Umsetzung von 3-Methylbut-3-en-1-ol der Formel (III) 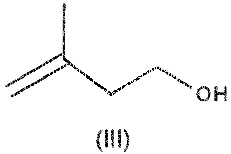 mit einem Aldehyd der Formel (IV)
R¹-CHO (IV)
wobei R¹ in der Formel (IV) die zuvor angegebene Bedeutung hat,
in Gegenwart eines sauren Katalysators, **dadurch gekennzeichnet, dass** die Umsetzung in einer Anordnung aus n in Reihe geschalteter Reaktoren erfolgt, wobei n für eine natürliche Zahl von wenigstens 2 steht, wobei zwischen dem in Strömungsrichtung ersten und letzten Reaktor einen Teilstrom entnommen und in einen stromaufwärts der Entnahmestelle gelegenen Reaktor eingespeist wird.

2. Verfahren nach Anspruch 1, **dadurch gekennzeichnet, dass** die Umsetzung kontinuierlich erfolgt.

3. Verfahren nach einem der Ansprüche 1 oder 2, **dadurch gekennzeichnet, dass** die Umsetzung in Gegenwart eines Lösungsmittels, insbesondere in Gegenwart von Wasser erfolgt.

4. Verfahren nach Anspruch 3, **dadurch gekennzeichnet, dass** dem Reaktoraustrag des in Strömungsrichtung ersten und/oder zweiten Reaktors ein Teilstrom entnommen und über eine externe Rückführung zumindest teilweise in den in Strömungsrichtung ersten Reaktor zurückgeführt wird.

5. Verfahren nach Anspruch 4, **dadurch gekennzeichnet dass** ein Teilstrom des Reaktoraustrags aus dem ersten Reaktor abgezogen und über einen externen Kreislauf in den in Strömungsrichtung ersten Reaktor zurückgeführt wird.

6. Verfahren nach einem der Ansprüche 4 bis 5, **dadurch gekennzeichnet, dass** dem Teilstrom vor der Einspeisung in einen stromaufwärts der Entnahmestelle gelegenen Reaktor Wärme entzogen wird.

7. Verfahren nach einem der vorhergehenden Ansprüche, **dadurch gekennzeichnet, dass** der in Strömungsrichtung (n-1)-te Reaktor weitgehend isotherm betrieben wird, wobei weitgehend isotherm betrieben bedeutet, dass das Temperaturintervall ΔT im Reaktor kleiner als die adiabatische Temperaturerhöhung ist.

8. Verfahren nach einem der vorhergehenden Ansprüche, **dadurch gekennzeichnet, dass** dem Reaktoraustrag aus mindestens einem der ersten bis (n-1)-ten Reaktoren vor Zuführung in den in Strömungsrichtung folgenden Reaktor Wärme entzogen wird.

9. Verfahren nach einem der vorhergehenden Ansprüche, **dadurch gekennzeichnet, dass** zumindest der in Strömungsrichtung letzte Reaktor ohne Rückführung des Reaktoraustrags betrieben wird.

10. Verfahren nach einem der vorhergehenden Ansprüche, **dadurch gekennzeichnet, dass** die Umsetzung zumindest in dem in Strömungsrichtung letzten Reaktor adiabatisch durchgeführt wird, wobei unter adiabatischer Reaktionsführung eine Vorgehensweise im technischen Sinne verstanden wird, bei der die bei der Reaktion freiwerdende Wärmemenge von der Reaktionsmischung im Reaktor aufgenommen und keine Kühlung durch Kühlvorrichtungen erfolgt, wodurch die Reaktionswärme weitestgehend mit dem Reaktionsgemisch aus dem Reaktor abgeführt und ein Restanteil durch natürliche Wärmeleitung bzw. -strahlung vom Reaktor an die Umgebung abgegeben wird.

11. Verfahren nach einem der vorhergehenden Ansprüche, **dadurch gekennzeichnet, dass** zur Umsetzung eine Reaktoranordnung verwendet wird, die wenigstens einen Festbettreaktor umfasst, vorzugsweise eine Reaktoranordnung eingesetzt wird, in der alle n Reaktoren Festbettreaktoren sind.

12. Verfahren nach einem der vorhergehenden Ansprüche, **dadurch gekennzeichnet, dass** zur Umsetzung eine Reaktoranordnung verwendet wird, die wenigstens einen Reaktor mit einem intern angeordneten Wärmeübertrager umfasst.

13. Verfahren nach einem der vorhergehenden Ansprüche, **dadurch gekennzeichnet, dass** der Rest R¹ für Isobutyl oder Phenyl steht.

14. Verfahren nach einem der vorhergehenden Ansprüche, **dadurch gekennzeichnet, dass** die Umsetzung in Gegenwart eines sauren Katalysators erfolgt, der vorzugsweise ausgewählt ist unter Salzsäure, Schwefelsäure, Methansulfonsäure, p-Toluolsulfonsäure und stark sauren Kationenaustauschern.

15. Verfahren nach einem der vorhergehenden Ansprüche, **dadurch gekennzeichnet dass** der Alkohol der Formel (III) und der Aldehyd der Formel (IV) in einem molaren Verhältnis im Bereich von 0,7 : 1 bis 2 : 1 eingesetzt werden.

## Claims

1. A process for the preparation of 2-substituted 4-hydroxy-4-methyltetrahydropyrans of the general formula (I) 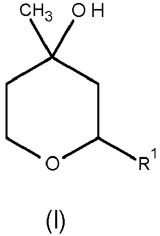 in which
R¹ is a straight-chain or branched C₁-C₁₂-alkyl, straight-chain or branched C₂-C₁₂-alkenyl, unsubstituted or C₁-C₁₂-alkyl and/or C₁-C₁₂-alkoxy substituted cycloalkyl having in total 3 to 20 carbon atoms or unsubstituted or C₁-C₁₂-alkyl and/or C₁-C₁₂-alkoxy substituted aryl having in total 6 to 20 carbon atoms,
comprising a reaction of 3-methylbut-3-en-1-ol of the formula (III) 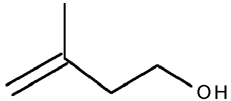 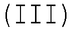 with an aldehyde of the formula (IV)
R¹-CHO (IV)
where R¹ in the formula (IV) has the meaning given above,
in the presence of an acidic catalyst, wherein the reaction takes place in an arrangement consisting of n reactors connected in series, n being a natural number of at least 2, wherein a part stream is removed between the first and last reactor in the flow direction and is fed into a reactor positioned upstream of the removal point.

2. The process according to claim 1, wherein the reaction takes place continuously.

3. The process according to either of claims 1 and 2, wherein the reaction takes place in the presence of a solvent, in particular in the presence of water.

4. The process according to claim 3, wherein a part stream is removed from the reactor discharge of the first and/or second reactor in the flow direction and is returned at least partially to the first reactor in the flow direction via an external recirculation.

5. The process according to claim 4, wherein a part stream of the reactor discharge is stripped off from the first reactor and is returned to the first reactor in the flow direction via an external circuit.

6. The process according to either of claims 4 to 5, wherein heat is withdrawn from the part stream before it is fed into a reactor positioned upstream of the removal point.

7. The process according to any one of the preceding claims, wherein the (n-1) th reactor in the flow direction is operated largely isothermally, wherein operated largely isothermally means that the temperature interval Δ T in the reactor is smaller than the adiabatic temperature increase.

8. The process according to any one of the preceding claims, wherein heat is withdrawn from the reactor discharge from at least one of the first to (n-1)th reactors before introducing it into the following reactor in the flow direction.

9. The process according to any one of the preceding claims, wherein at least the last reactor in the flow direction is operated without recirculation of the reactor discharge.

10. The process according to any one of the preceding claims, wherein the reaction at least in the last reactor in the flow direction is carried out adiabatically, wherein adiabatic reaction implementation is understood as meaning a procedure in the technical sense in which the amount of heat that is released during the reaction is absorbed by the reaction mixture in the reactor and no cooling takes place by means of cooling devices, as a result of which the heat of reaction is substantially removed from the reactor with the reaction mixture and a residual amount is released into the surroundings as a result of natural heat conduction and/or radiation from the reactor.

11. The process according to any one of the preceding claims, wherein a reactor arrangement is used for the reaction which comprises at least one fixed-bed reactor, preferably a reactor arrangement in which all n reactors are fixed-bed reactors.

12. The process according to any one of the preceding claims, wherein a reactor arrangement is used for the reaction which comprises at least one reactor with an internally arranged heat exchanger.

13. The process according to any one of the preceding claims, wherein the radical R¹ is isobutyl or phenyl.

14. The process according to any one of the preceding claims, wherein the reaction takes place in the presence of an acidic catalyst which is preferably selected from hydrochloric acid, sulfuric acid, methanesulfonic acid, p-toluenesulfonic acid and strongly acidic cation exchangers.

15. The process according to any one of the preceding claims, wherein the alcohol of the formula (III) and the aldehyde of the formula (IV) are used in a molar ratio in the range from 0.7:1 to 2:1.

## Revendications

1. Procédé de fabrication de 4-hydroxy-4-méthyl-tétrahydropyranes substitués en position 2 de formule générale (I) 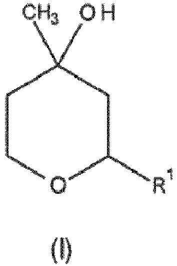 dans laquelle
R¹ représente alkyle en C₁-C₁₂ linéaire ou ramifié, alcényle en C₂-C₁₂ linéaire ou ramifié, cycloalkyle non substitué ou substitué avec alkyle en C₁-C₁₂ et/ou alcoxy en C₁-C₁₂, contenant au total 3 à 20 atomes de carbone, ou aryle non substitué ou substitué avec alkyle en C₁-C₁₂ et/ou alcoxy en C₁-C₁₂, contenant au total 6 à 20 atomes de carbone,
comprenant une mise en réaction de 3-méthylbut-3-én-1-ol de formule (III) 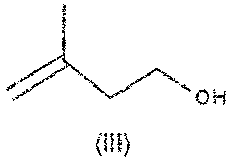 avec un aldéhyde de formule (IV)
R¹-CHO (IV)
R¹ dans la formule (IV) ayant la signification indiquée précédemment,
en présence d'un catalyseur acide, **caractérisé en ce que** la réaction a lieu dans un agencement de n réacteurs raccordés en série, n représentant un nombre naturel d'au moins 2, un courant partiel étant soutiré entre le premier et le dernier réacteur dans la direction de l'écoulement, et introduit dans un réacteur situé en amont de l'emplacement de soutirage.

2. Procédé selon la revendication 1, **caractérisé en ce que** la réaction a lieu en continu.

3. Procédé selon l'une quelconque des revendications 1 ou 2, **caractérisé en ce que** la réaction a lieu en présence d'un solvant, notamment en présence d'eau.

4. Procédé selon la revendication 3, **caractérisé en ce qu'**un courant partiel est soutiré à partir de la sortie de réacteur du premier et/ou du deuxième réacteur dans la direction de l'écoulement, et recyclé au moins en partie par une conduite de recyclage externe dans le premier réacteur dans la direction de l'écoulement.

5. Procédé selon la revendication 4, **caractérisé en ce qu'**un courant partiel est soutiré à partir de la sortie de réacteur du premier réacteur et recyclé dans le premier réacteur dans la direction de l'écoulement par un circuit externe.

6. Procédé selon l'une quelconque des revendications 4 à 5, **caractérisé en ce que** de la chaleur est extraite du courant partiel avant l'introduction dans un réacteur situé en amont de l'emplacement de soutirage.

7. Procédé selon l'une quelconque des revendications précédentes, **caractérisé en ce que** le réacteur (n-1) dans la direction de l'écoulement est exploité sous forme essentiellement isotherme, exploité sous forme essentiellement isotherme signifiant que l'intervalle de température ΔT dans le réacteur est inférieur à l'élévation de température adiabatique.

8. Procédé selon l'une quelconque des revendications précédentes, **caractérisé en ce que** de la chaleur est extraite de la sortie de réacteur d'au moins un des réacteurs compris entre le premier réacteur et le réacteur (n-1) avant l'introduction dans le réacteur suivant dans la direction de l'écoulement.

9. Procédé selon l'une quelconque des revendications précédentes, **caractérisé en ce qu'**au moins le dernier réacteur dans la direction de l'écoulement est exploité sans recyclage de la sortie de réacteur.

10. Procédé selon l'une quelconque des revendications précédentes, **caractérisé en ce que** la réaction au moins dans le dernier réacteur dans la direction de l'écoulement est réalisée sous forme adiabatique, une réalisation sous forme adiabatique de la réaction étant comprise au sens technique comme une procédure selon laquelle la quantité de chaleur libérée lors de la réaction est absorbée par le mélange réactionnel dans le réacteur et aucun refroidissement n'a lieu par des dispositifs de refroidissement, la chaleur de réaction étant ainsi très largement déchargée du réacteur avec le mélange réactionnel et une fraction résiduelle étant émise dans l'environnement par conduction ou rayonnement de chaleur naturel depuis le réacteur.

11. Procédé selon l'une quelconque des revendications précédentes, **caractérisé en ce qu'**un agencement de réacteurs qui comprend au moins un réacteur à lit fixe est utilisé pour la réaction, de préférence un agencement de réacteurs dans lequel les n réacteurs sont tous des réacteurs à lit fixe.

12. Procédé selon l'une quelconque des revendications précédentes, **caractérisé en ce qu'**un agencement de réacteurs qui comprend au moins un réacteur contenant un échangeur de chaleur agencé en interne est utilisé pour la réaction.

13. Procédé selon l'une quelconque des revendications précédentes, **caractérisé en ce que** le radical R¹ représente isobutyle ou phényle.

14. Procédé selon l'une quelconque des revendications précédentes, **caractérisé en ce que** la réaction a lieu en présence d'un catalyseur acide, qui est de préférence choisi parmi l'acide chlorhydrique, l'acide sulfurique, l'acide méthane-sulfonique, l'acide p-toluène-sulfonique et les échangeurs de cations acides forts.

15. Procédé selon l'une quelconque des revendications précédentes, **caractérisé en ce que** l'alcool de formule (III) et l'aldéhyde de formule (IV) sont utilisés en un rapport molaire dans la plage allant de 0,7:1 à 2:1.
